# EUROPEAN PATENT SPECIFICATION

(11) **EP 1 724 578 B1**
(45) Date of publication and mention of the grant of the patent: **11.07.2018**
(21) Application number: 06075958.6
(22) Date of filing: 27.04.2006
(51) Int. Cl.: G01N 33/46, G01N 29/44, G01N 29/07

(54) **Non-destructive analysis system for wooden objects**
Zerstörungsfreies Analysesystem für Holzgegenstände
Système d'analyse non destructif des objets en bois

(30) Priority: 28.04.2005 NL 1028895
(43) Date of publication of application: 22.11.2006
(73) Proprietor: Brookhuis Applied Technologies B.V., 7500 AB Enschede (NL)
(72) Inventor: Elbers, Martien Cornelis Maria, 7559 CV Hengelo (NL); Rozema, Pieter, 7577 MW Oldenzaal (NL)
(74) Representative: EP&C

(56) References cited:
- EP-A- 0 763 344
- US-A- 5 404 755
- US-A- 6 139 496
- US-A1- 2004 035 208
- US-B1- 6 347 551
- US-B1- 6 813 927

## Description

The invention relates to a non-destructive object analysis system for the analysis of wooden objects.

US 6,813,927 discloses a system for the non-destructive analysis of a wooden object on the basis of the principle that the user hits the end of a wooden beam with a hammer and thus subjects the wooden beam to a single impulse. Herein the reflection of said impulse is received by a sensor which is held by the user with his other hand against the same end of the wooden beam as onto which the hammer blow has been exerted. The signal of this sensor is than processed with suitable signal processing means to one or more analysis results which relate to the wooden object that has been analysed. This method of analysis is based on the relation in a solid material between the propagation speed of waves in said material and the density and modulus of elasticity of the material.

In the wood processing industry it is known to determine the strength of felt trees, wooden planks, beams, etc., but also of end products made of wood, such as building constructions and construction elements on the basis of the principle described above. For example Goerlacher, "Sortierung von Brettschichtholzlamellen nach DIN 4074 durch Messung von Longitudinalschwingungen", Nauingenieur vol. 65, 1990 describes an analysis of timber on the basis of the above principle. In this manner also for example the quality of glued connections in timber constructions can be analysed. The principle is also used to determine decay in wooden objects, for example in trees but also in wooden railway sleepers.

The known system described above is not satisfactory in practice. It has been found time-consuming to perform a large number of analyses - as is often required in the timber industry - in particular when the wooden objects to be examined are placed at different locations. In addition it has been found that non-experienced users have problems to perform a reliable analysis, in particular as one has the inclination to hit hard and uncontrolled with the hammer on the wooden object. This problem is already described in US 6,813,927 and the solution proposed therein is to provide the analysis apparatus in combination with a hammer of a certain weight. In this manner at least not a different hammer (with a different weight) is used all the time.

Also known are stationary mounted in-line systems, wherein the wood is guided along a stationary measuring device with a hammer and a sensor. With such an in-line system a large number of analysis can be performed but as a result of the stationary arrangement of the system and the necessary transportation of the wood to said system, this system too is time consuming and in addition inflexible when used.

US2004/0035208 discloses portable ultrasound inspection apparatus embodied as a handheld pistol. On the basis of ultrasound a material, e.g. the content of a container, can be identified. The transducer assembly of the apparatus contains a high frequency transducer and a low frequency transducer.

US5404755, on which the preamble of claim 1 is based, disclose an apparatus with a carriage having rollers and one or more hammers to generate an impulse. The apparatus comprises a stem to carry and hold the apparatus by hand.

The present invention has as an object to provide an improved system, at least has as an object to provide a usable alternative for the known systems.

The invention achieves the object mentioned above by providing a system according to claim 1.

In the system according to claim 1 it is provided for that in the portable housing both the impulse generator and the sensor, which receives the reflection of the generated impulse, are mounted.

The impulse generator is adapted to generate a single calibrated impulse, essentially corresponding to a blow with a hammer, such that the measurements with the system are reproducible.

Compared to the prior art system as described earlier in US 6,813,927 it is achieved that the user is less strained (he does not have to hit with a hammer anymore himself) and can in this manner perform more analysis.

The impulse generator is provided with electrical power means for generating the calibrated impulse, for example with an electromagnetically movable impulse element which copies the blow with a hammer.

The portable apparatus is adapted to be held and carried with a single hand during the performance of an analysis, wherein then the portable apparatus is held against the wooden object to be analysed with said one hand. This has the advantage that the user has his other hand free, for example for handling the object to be analysed, for example a wooden beam or plank.

The housing of the user portable apparatus comprises a stem which can be gripped with a single hand and furthermore a head aligned with said stem, outside the area where the hand grips the stem. Herein the active side of the housing is formed by a surface of said head, said surface at the end of the head remote from the stem and essentially at right angles to a longitudinal axis of the stem. By this ergonomically advantageous embodiment the user can hold the apparatus stable and also hold it against the object to be analysed, possibly press it against the object.

In the embodiment described above it is advantageous if the display means comprise a display which is mounted in the head so that it is visible for the user.

In an advantageous embodiment the signal processing means are mounted within the housing of the user portable apparatus. For instance a suitable microprocessor is provided, which on the basis of a software program deducts one ore more analysis results from the signal obtained by the sensor. Preferably it is envisaged that a software program dedicated for the material to be analysed is used, for example a program for multiple materials having a selection possibility for the user.

In an advantageous embodiment the display means are mounted in the housing of the user portable apparatus, such as a display, for example a LCD-display.

It will be apparent that with special preference both the signal processing means and the display means are mounted in the portable apparatus. This results in a portable and autonomous and fully functional analysis apparatus.

Preferably wireless communication means are mounted within the housing of the user portable apparatus, such that one or more components in the portable apparatus, for instance the sensor and/or the processing means, can communicate with remote equipment, for example with a computer, such as for example a laptop computer.

For example, the wireless communication means are adapted for establishing a radio connection, for example according to the Bluetooth standard as is described by the Bluetooth-SIG.

The housing of the portable apparatus is provided with one or more control members, which are at least adapted for activating the impulse generator.

As the portable apparatus is adapted to be held with a single hand during the performance of an analysis it is advantageous if a control member which activates the impulse generator can be controlled with the same hand as with which the portable apparatus is held, preferably with the thumb of said hand. This allows holding the apparatus stable and at the same time easy to activate the impulse generator, for instance by pressing a specific push button.

In a possible embodiment the system further comprises a computer, for instance a laptop computer, which is separate from the portable apparatus, wherein possibly a wireless connection is provided between the portable apparatus and the computer. As is mentioned before an embodiment can be envisaged wherein the processing of the sensor signal to one or more analysis results does not take place in the portable apparatus. In said version it is advantageous to realise said processing in a separate computer, preferable a portable computer. By establishing a wireless communication between said apparatus and the computer problems related to a cable connection are prevented. Such problems include the risk of tripping over the cable (possibly with damage to the cable and/or the apparatus and/or the computer), damage to the cable (for instance by wooden beams falling thereon), limited range of the apparatus which respect to the computer, etc.

If the signal processing to one or more analysis results takes place in the computer, it can be envisaged that the results are transmitted back to the apparatus (possible wireless), such that they are visible there for the user. Obviously the results can also be presented on a display of the computer.

In another version wherein the system includes a computer the signal processing means are positioned within the portable apparatus with therein the impulse generator and the sensor, but is than possible to transmit the one or more analysis results to the computer, for example for storage in order for preparation of subsequent reports, etc.

In an advantageous embodiment it is envisaged that the portable apparatus with therein the impulse generator and the sensor, and preferably also the single processing means, is provided with a memory wherein a (large) number of data, in particular analysis results, can be stored. Preferably in connection with wireless communication means in the portable apparatus, it is for example envisaged that the data to be stored in the memory are transmitted to a computer for further purposes, such as for logging, preparation of reports, further (statistical) processing, etc.

When conducting a non-destructive analysis of the type described herein it can be advantageous that the weight of the object to be analysed is determined. Said weight can then possibly in combination with the one or more analysis results be displayed and possible be stored, but it can also be envisaged that the weight forms a parameter for the signal processing which is carried out on the basis of the sensor signal.

For the situation mentioned above the invention envisages that the system further includes a weighing device for weighing the object to be analysed.

In a possible embodiment it is envisaged that the signal processing means which process the sensor signal are also adapted to receive a weighing signal of the weighing device. As mentioned said weighing signal could be displayed along with the one ore more analyses results and/or be stored, or be incorporated into and have an influence on to the one or more analysis results.

The weighing device can include one or possibly several weighing units, for example two weighing units if the weight of long objects (for example wooden beams) has to be established. For example the one or more weighing units are provided with a platform onto which the object to be weighed is placed.

Preferably it is envisaged that a weighing device composed of one or more weighing units is provided with wireless communication means, such a weighing device can transfer the weighing signal in a wireless manner to for example the portable apparatus with the impulse generator and the sensor therein and/or to a computer belonging to the system.

For instance the signal processing means are mounted in the portable apparatus and said portable apparatus already being provided with wireless communication means to communicate with a computer it is then advantageous if the weighing device can use the same wireless communication means to communicate with the portable apparatus or the computer, for instance on the basis of the Bluetooth system.

Concerning a system provided with a weighing device it is noted that the idea to provide the weighing device with wireless communication means can also be applied to prior art analysis systems of the type wherein an impulse generator and sensor are not mounted within a single common housing, but for instance comprises a hammer and a separate sensor.

In a practical embodiment the portable apparatus with the impulse generator and the sensor therein is provided with one or more batteries, for example one or more rechargeable batteries.

Advantageously the signal processing means are adapted for providing an analysis result that is related to the strength of the analysed wooden object.

The invention will hereinafter be described in more detail referring to the drawing of a non-limitative example of the system according to the invention. In the drawings:
Fig. 1 shows in perspective view an apparatus according to the invention which can be held and carried with a single hand;
Fig. 2 shows very schematically the apparatus and the components mounted therein;
Fig. 3 shows the apparatus of fig. 1 when an analysis of an object is performed;
Fig. 4 shows schematically a system with the apparatus of figs. 1-3, and also a computer and wireless communication;
Fig. 5 shows schematically an alternative of the system of fig. 4, wherein furthermore a weighing device is provided for the object to be analysed.

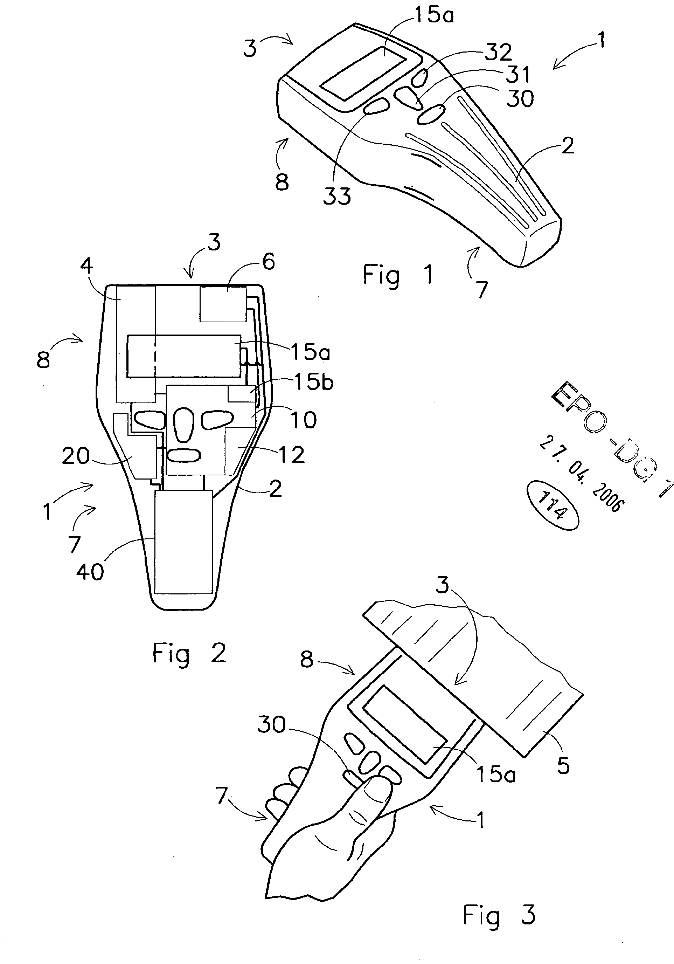

The figures 1-3 show a non-destructive object analyses system, which is here embodied as a single fully functional apparatus 1, wherein the apparatus 1 is adapted to be held and carried with a single hand during the performance of an analysis.

In the description which follows the apparatus 1 is used for the analyses of wooden objects, such as in the timber industry, in particular for the determination of the class of strength and/or modulus of elasticity of each wooden object.

In the timber industry it is desired to sort wood in classes of strength, in particular when the wood is used for structures which are mechanically loaded. The apparatus 1 allows the user to analyse a large number of wooden objects fast and efficiently under ergonomically favourable conditions.

The apparatus 1 comprises in this example a housing, preferably a dust- and/or watertight housing 2 with an active side 3. In the housing 2 an impulse generator 4 is mounted such that when the user holds the housing 2 with its active side 3 against the object to be analysed (for example the wooden beam 5 in fig. 3) the impulse generator 4 can subject the wooden object to a single calibrated impulse.

In the housing 2, here at the active side 3, a sensor 6 is mounted which is adapted to receive a reflection of the impulse which has been passed to the object by the impulse generator 4 and for providing a sensor signal.

Preferably the sensor 6 is an acceleration sensor, for example a piezo-electrical sensor, but other sensor embodiments, which can sense a vibration, such as a microphone, pressure sensor or laser sensor are also possible.

In the housing 2 furthermore signal processing means 10 are mounted which are connected to the sensor 6 and serve to process the sensor signal into one ore more analysis results. These signal processing means 10 for example comprise a microprocessor and an associated software program, which is preferably specifically designed for wood.

The software and/or other means to adjust the setting of the apparatus to the object to be analysed preferably are mounted within the handheld apparatus itself. Herewith the settings can thus be adapted locally to the object and a suitable flexibility of use is provided.

In the housing 2 furthermore display means are mounted for displaying the one or more analysis results provided by the processing means 10. In the embodiment shown here the display means comprise a display 15a and associated control circuitry 15b, such as a LCD-display 15a.

In the housing 2 furthermore wireless communication means 20 are mounted. For example these wireless communication means 20 act on the basis of a radio connection, preferably according to the Bluetooth-standard.

In an embodiment not shown here - as an alternative or in addition - also a connection for a solid state data transmission can be mounted on the apparatus 1. Herewith the apparatus can be connected to a computer via a communication cable, for example when the wireless communication means have broken down or are not necessary.

The housing 2 of the portable apparatus 1 is furthermore provided with several control members 30-33, for example for switching the apparatus 1 on or off, operating the software of the signal processing means, operating the communication, and for the activation of the impulse generator 4.

In this example the control member 30, here embodied as a push button, activates the impulse generator 4. This push button 30 is operable with the same hand which carries the portable apparatus 1, preferably with the thumb of said hand as is shown in fig. 3.

In the housing 2 furthermore one or more electrical batteries 40 are received or can be placed, for example a rechargeable battery 40. The electrical energy in the battery 40 feeds all electrical components.

Preferably the apparatus 1 is provided with a connection (not shown) for an external electrical power supply, such as the apparatus can also be used when the battery 40 is empty or defective.

In fig. 3 it can be recognised that the housing 2 comprises a stem 7, which can be gripped with a single hand, and furthermore a head 8 at the end of said stem 7, wherein the active side 3 of the housing 2 is formed by a surface of said head 8, in particular a surface at the end of the head 8 which is remote from the stem, which surface 4 is essentially at right angles to a longitudinal axis of the stem 7.

In fig. 2 it is shown that the battery is received in the stem 7. This provides a favourable weight distribution in the apparatus, which is advantageous from an ergonomic point of view.

The display 15a is located in an upper surface of the head 8, in this example at the same side as the one or more control members 30-33 which can be operated with the thumb.

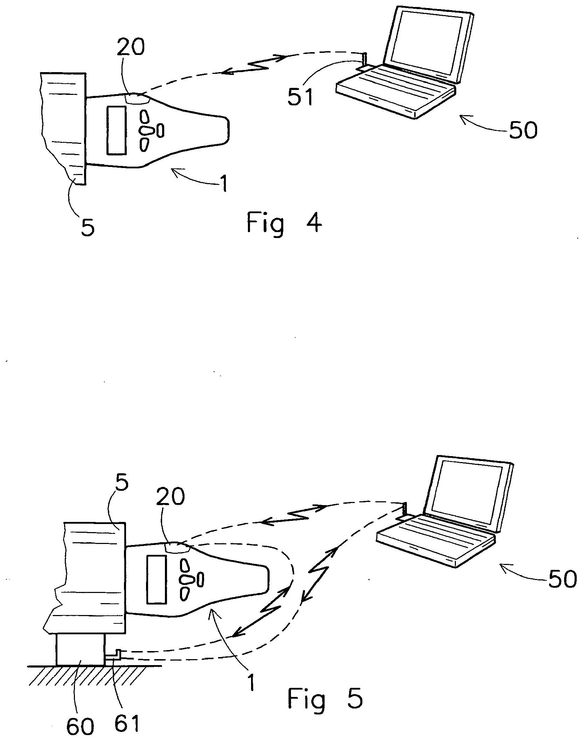

In fig. 4 the system comprises not only the apparatus 1 but also a computer 50, in this example a laptop computer, which is separate from the portable apparatus 1. In this example it is also provided for that a wireless communication exists between the portable apparatus 1 and the computer 50, preferably on the basis of the Bluetooth system, for example with a suitable insert card with antenna 51.

In an advantage embodiment the apparatus 1 is also provided with a memory 12 for storage of a (large) number of analysis results. These results can then possibly on the basis of the wireless communication be transmitted to the computer 50, for example for logging, drafting reports and the like.

Preferably the signal processing with respect to for example identification of a shipment of timber, which could for example comprise details of the origin, destination, specific properties, applicable standard, etc., is carried out on the computer as it contains a complete keyboard.

In a version which has not been shown the signal processing means 10 and/or the memory 12 are absent in the apparatus 1, and these means are provided for in the computer 50. In this embodiment the sensor signal could possibly be transmitted to the computer.

If the processing of the signal into one or more analyses results takes place in the computer 50 it can be envisaged that the results are transmitted back (preferably wireless) to the apparatus 1, such that these are visible for the user on the display 15a. Obviously the results can also be presented on a display of the computer 50.

The signal processing means 10 which have been arranged in the housing of the apparatus 1 or in the computer are adapted such that reliable, robust and reproducible analysis results are obtained. Such analysis results are for instance important in a production quality control system.

In the system according to fig. 5 it is further envisaged that the weight of the object to be analysed can be determined by means of a weighing device.

In the example the weighing device 60 comprises a single weighing unit but an assembly of several (for example two) weighing units is also possible.

The one or more weighing units are here provided with wireless communication means 61, such that the weighing units can communicate in said manner with the apparatus 1 and/or the computer 50, for example on the bases of the same Bluetooth system. Herein in each weighing unit is preferably provided with its own battery (rechargeable) for feeding the electrical weighing sensor and the circuitry which provides the signal as well as the wireless communication means 61.

The impulse generator 4 preferably is electrically powered, such that upon operation of the button 30 an electrical current is fed to the generator 4 and a single impulse is released. The impulse essentially corresponds to the blow of a hammer, such as can be exerted by a user with a hammer which is common in the industry for this type of measurement. For example an electromagnetically movable impulse element is provided for, for example an element which can be displaced under the influence of a current through a surrounding coil and such can exert an impulse on the wooden object.

In practice the analysis results obtained with the system according to the invention can be complemented with other results, for example a length measurements of the object (which could possible an input parameter for the signal processing of the reflection of the impulse), a wood moisture measurement, a measurement of the width and/or thickness, a measurement of the weight, a measurement of the specific weight, and the like. These measurement results could be obtained with suitable measuring devices which could like the weighing device described herein be connected to the system either wireless or not.

## Claims

1. Non-destructive wooden object analysis system comprising:
- a user portable apparatus (1) which is adapted to be held and carried with a single hand during the performance of an analysis , which apparatus comprises:
- a housing (2) comprising a stem (7) having a longitudinal axis, which stem can be gripped with a single hand, said housing having an active side (3) with which the user holds the apparatus - when in use - against the wooden object to be analysed,
- a sensor (6), which is mounted in the housing and which is adapted to sense a reflection of an impulse transmitted to the object and which is adapted for providing a sensor signal,
- signal processing means (10) for processing the sensor signal and providing one or more analysis results,
- display means (15a, b; 50) for displaying the one or more analysis results,
wherein the housing of the portable apparatus is provided with one or more control members (30-33), which are at least adapted to activate the impulse generator (4),
wherein in the housing (2) of the portable apparatus (1) furthermore an impulse generator (4) is mounted, which impulse generator (4) is provided with electrical power means adapted to generate a single calibrated impulse, essentially corresponding to a blow with a hammer, and transfer thereof to the wooden object when the user holds the housing (2) with its active side (3) against the object (5) to be analysed,
**characterised in that**
the housing furthermore comprises a head (8) that is aligned with said stem (7), outside the area where the hand grips the stem,
and **in that** the active side of the housing is formed by a surface of said head (8), said surface being located at the end of the head (8) remote from the stem (7),
and **in that** the surface forming the active side of the housing is essentially at right angles to the longitudinal axis of the stem (7).

2. System according to one or more of the preceding claims, wherein a control member (30) which activates the impulse generator (4) is operable with the same hand which is used to carry and hold the portable apparatus (1).

3. System according to one or more of the preceding claims, wherein the signal processing means (10) are mounted in the housing of the user portable apparatus (1).

4. System according to one or more of the preceding claims, wherein wireless communication means (20) are mounted in the housing of the user portable apparatus.

5. System according to one or more preceding claims, wherein the display means comprise a display (15a), which is mounted visible in the head (8).

6. System according to one or more of the preceding claims, wherein the system furthermore comprises a computer (50), which is separate from the portable apparatus (1), wherein a wireless communication (20, 51) is provided between the portable apparatus and the computer.

7. System according to one or more of the preceding claims, wherein the system furthermore comprises a weighing device (60) for weighing the object to be analysed, wherein the signal processing means are adapted for receiving a weighing signal from the weighing device and determination of the one or more analysis results also on the basis of said weighing signal.

## Patentansprüche

1. System zur zerstörungsfreien Analyse eines Holzgegenstands, umfassend:
- eine von einem Benutzer tragbare Vorrichtung (1), welche ausgestaltet ist, um während der Ausführung einer Analyse mit einer Hand gehalten und getragen zu werden, wobei die Vorrichtung umfasst:
- ein Gehäuse (2), welches einen Schaft (7) mit einer Längsachse umfasst, wobei der Schaft mit einer Hand gegriffen werden kann, wobei das Gehäuse eine aktive Seite (3) aufweist, mit welcher der Benutzer die Vorrichtung - wenn sie sich im Betrieb befindet - gegen den zu analysierenden Holzgegenstand hält,
- einen Sensor (6), welcher in dem Gehäuse montiert ist und welcher ausgestaltet ist, um eine Reflektion eines Impulses zu erfassen, der zu dem Objekt übertragen wird, und welcher ausgestaltet ist, um ein Sensorsignal bereitzustellen,
- Signalverarbeitungsmittel (10), um das Sensorsignal zu verarbeiten und um ein oder mehrere Analyseergebnisse bereitzustellen,
- Anzeigemittel (15a, b; 50), um das eine oder die mehreren Analyseergebnisse darzustellen,
wobei das Gehäuse der tragbaren Vorrichtung mit einem oder mit mehreren Steuerteilen (30-33) versehen ist, welche zumindest ausgestaltet sind, um den Impulsgenerator (4) zu aktivieren,
wobei in dem Gehäuse (2) der tragbaren Vorrichtung (1) darüber hinaus ein Impulsgenerator (4) montiert ist, wobei der Impulsgenerator (4) mit elektrischen Energiemitteln versehen ist, welche ausgestaltet sind, um einen einzigen kalibrierten Impuls zu erzeugen, welcher im Wesentlichen einem Schlag mit einem Hammer entspricht, und diesen zu dem Holzgegenstand zu übertragen, wenn der Benutzer das Gehäuse (2) mit seiner aktiven Seite (3) gegen das zu analysierende Objekt (5) hält,
**dadurch gekennzeichnet,**
**dass** das Gehäuse darüber hinaus einen Kopf (8) außerhalb des Bereiches, wo die Hand den Schaft greift, umfasst, welcher mit dem Schaft (7) ausgerichtet ist, und dass die aktive Seite des Gehäuses durch eine Oberfläche des Kopfes (8) ausgebildet ist, wobei die Oberfläche an dem Ende des Kopfes (8) abgesetzt von dem Schaft (7) angeordnet ist,
und **dass** die Oberfläche, welche die aktive Seite des Gehäuses ausbildet, im Wesentlichen rechte Winkel zu der Längsachse des Schafts (7) aufweist.

2. System nach einem oder mehreren der vorhergehenden Ansprüche, wobei ein Steuerteil (30), welches den Impulsgenerator (4) aktiviert, mit derselben Hand betriebsbereit ist, welche eingesetzt wird, um die tragbare Vorrichtung (1) zu tragen und zu halten.

3. System nach einem oder mehreren der vorhergehenden Ansprüche, wobei die Signalverarbeitungsmittel (10) in dem Gehäuse der von dem Benutzer tragbaren Vorrichtung (1) montiert sind.

4. System nach einem oder mehreren der vorhergehenden Ansprüche, wobei drahtlose Kommunikationsmittel (20) in dem Gehäuse der von dem Benutzer tragbaren Vorrichtung montiert sind.

5. System nach einem oder mehreren der vorhergehenden Ansprüche, wobei die Anzeigemittel eine Anzeige (15a) umfassen, welche sichtbar in dem Kopf (8) montiert ist.

6. System nach einem oder mehreren der vorhergehenden Ansprüche, wobei das System darüber hinaus einen Computer (50) umfasst, welcher getrennt von der tragbaren Vorrichtung (1) ist, wobei eine drahtlose Kommunikation (20, 51) zwischen der tragbaren Vorrichtung und dem Computer bereitgestellt wird.

7. System nach einem oder mehreren der vorhergehenden Ansprüche, wobei das System darüber hinaus eine Wiegevorrichtung (60) umfasst, um das zu analysierende Objekt zu wiegen, wobei die Signalverarbeitungsmittel ausgestaltet sind, um ein Wiegesignal von der Wiegevorrichtung zu empfangen und das eine oder die mehreren Analyseergebnisse auch auf der Basis des Wiegesignals zu bestimmen.

## Revendications

1. Système d'analyse non-destructive d'un objet en bois comprenant :
- un appareil portable par un utilisateur (1) qui est adapté pour être tenu et porté avec une seule main pendant une analyse, ledit appareil comprenant :
- un boîtier (2) comprenant une tige (7) ayant un axe longitudinal, ladite tige pouvant être attrapée avec une seule main, ledit boîtier ayant un côté actif (3) avec lequel l'utilisateur tient l'appareil - pendant son utilisation-contre l'objet en bois à analyser,
- un capteur (6), qui est monté dans le boîtier et qui est adapté pour détecter une réflexion d'une impulsion transmise à l'objet et qui est adapté pour fournir un - signal de capteur,
- un moyen de traitement de signal (10) destiné à traiter le signal de capteur et à fournir un ou plusieurs résultat(s) d'analyse,
- un moyen d'affichage (15a, b ; 50) destiné à afficher le ou les résultat(s) d'analyse,
dans lequel le boîtier de l'appareil portable est muni d'un ou plusieurs élément(s) de commande (30-33), qui est/sont au moins adapté(s) pour activer le générateur d'impulsion (4), dans lequel, dans le boîtier (2) de l'appareil portable (1), de plus, un générateur d'impulsion (4) est monté, ledit générateur d'impulsion (4) étant muni d'un moyen d'alimentation électrique adapté pour générer une seule impulsion calibrée, correspondant essentiellement à un coup de marteau, et pour transférer celle-ci à l'objet en bois lorsque l'utilisateur tient le boîtier (2) avec son côté actif (3) contre l'objet (5) à analyser,
**caractérisé en ce que**
le boîtier comprend en outre une tête (8) qui est alignée avec ladite tige (7), à l'extérieur de la zone dans laquelle la main attrape la tige,
et **en ce que** le côté actif du boîtier est formé par une surface de ladite tête (8), ladite surface étant située à l'extrémité de la tête (8) distante de la tige (7),
et **en ce que** la surface qui forme le côté actif du boîtier est essentiellement à des angles droits par rapport à l'axe longitudinal de la tige (7).

2. Système selon une ou plusieurs des revendications précédentes, dans lequel un élément de commande (30) qui active le générateur d'impulsion (4) peut être utilisé avec la même main que celle utilisée pour porter et tenir l'appareil portable (1).

3. Système selon une ou plusieurs des revendications précédentes, dans lequel le moyen de traitement de signal (10) est monté dans le boîtier de l'appareil portable par un utilisateur (1).

4. Système selon une ou plusieurs des revendications précédentes, dans lequel un moyen de communication sans fil (20) est monté dans le boîtier de l'appareil portable par un utilisateur.

5. Système selon une ou plusieurs des revendications précédentes, dans lequel le moyen d'affichage comprend un écran (15a), qui est monté de manière visible dans la tête (8) .

6. Système selon une ou plusieurs des revendications précédentes, dans lequel le système comprend en outre un ordinateur (50), qui est distinct de l'appareil portable (1), dans lequel une communication sans fil (20, 51) est prévue entre l'appareil portable et l'ordinateur.

7. Système selon une ou plusieurs des revendications précédentes, dans lequel le système comprend en outre un dispositif de pesée (60) destiné à peser l'objet à analyser, dans lequel le moyen de traitement de signal est adapté pour recevoir un signal de pesée de la part du dispositif de pesée et pour déterminer le ou les résultats(s) d'analyse également sur la base dudit signal de pesée.
